# EUROPEAN PATENT APPLICATION

(11) **EP 2 660 231 A1**
(43) Date of publication of application: **06.11.2013**
(21) Application number: 12166643.2
(22) Date of filing: 03.05.2012
(51) Int. Cl.: C07C 29/80, C07C 31/04, B01D 3/14

(54) **Process and separation column for separation of methanol**

(71) Applicant: Akzo Nobel Chemicals International B.V., 3811 MH Amersfoort (NL)
(72) Inventor: Kiss, Anton Alexandru, 6825 CM Arnhem (NL)
(74) Representative: De Vries, Adriaan Jacobus

(57) **Abstract**

A process for the purification of methanol wherein a raw grade methanol comprising methanol, lights and heavies is fed to a Petlyuk column installation (1, 20, 40), such as a dividing wall column. The Petlyuk column installation comprises a fractionation section (7, 25, 42) and a prefractionation section (6, 4, 41) isolated from the fractionation section. The raw grade methanol is fed to the prefractionation section. At least the largest part of the methanol content is passed along a top section (4, 22, 52) of the fractionation section where it is separated from the lights to form a pure grade methanol fraction.

## Description

The present invention relates to a process and a separation column for the separation of methanol, particularly from a mixture with water, heavy and light components.

In current practice methanol is typically produced from syngas, which in turn is produced by the partial combustion of natural gas or other hydrocarbon resources. Methanol is formed by the reaction of carbon monoxide with hydrogen:

CO + 2 H₂ → CH₃OH

During this process, CO₂ can be added to improve the C/H ratio.

Methanol can also be produced directly from CO₂ and H₂, without the intermediate production of syngas:

CO₂ + 3H₂ → CH₃OH + H₂O

Crude methanol typically contains water and various types of contaminants and impurities, including light and heavy hydrocarbon compounds. Hydrocarbon compounds having a lower boiling point than methanol are referred to as "lights", while hydrocarbon compounds having a higher boiling point than methanol are referred to as "heavies". Generally, the heavies stream mainly comprises water, and typically also includes higher alcohols, long-chain hydrocarbons and waxes, higher alkyl esters, higher ketones and esters of lower alcohols with formic, acetic or propionic acids. The lights typically include dissolved gases, dimethyl ether, methyl formate and acetone. The amount and composition of the impurities depend on reaction conditions during the methanol production, the feed gas, and the type of catalyst. Small amounts of caustic soda can also be present to neutralize lower carboxylic acids.

To isolate methanol from the light and heavy fractions, the crude methanol can be distilled. The various distillation steps require high energy consumption.

CN102190559 discloses a process for the treatment of methanol produced from syngas using a dividing top wall column and a pressurized rectification column. Before the crude methanol is fed to the dividing wall column, it is preheated in a heat exchanger. In the dividing wall column lights are collected at the top of the inlet side of the dividing wall column, while heavies are collected at the base of the column. A mixture of methanol, water and hydrocarbon impurities is collected laterally from the effluent side of the column and transported to the rectification column for purification. Further methanol fractions are separated near the top. This multi-step process is a complicated process requiring a relatively large number of equipment units. The obtained methanol fraction still contains more than 0.05 wt% of contaminants.

It is an object of the invention to provide a cost-effective process requiring less energy and less investment costs and enabling to yield methanol of a high purity grade.

The object of the invention is achieved with a process for the purification of methanol wherein a raw grade methanol is fed to a Petlyuk column installation comprising a fractionation section and a prefractionation section isolated from the fractionation section, wherein the raw grade methanol is fed to the prefractionation section and wherein at least the largest part of the methanol content is passed along a top section of the fractionation section to be subsequently discharged as a pure grade methanol fraction.

A Petlyuk column installation is a column arrangement for separating three or more components from a multi-component feed using a single reboiler to boil liquid from the bottom of the fractionation column to generate vapors which are returned to the column to drive the distillation separation, and one or more condensers for wholly or partially condensing vapours discharged from a top section of the fractionation column. Part of the condensed vapor may be returned to the fractionation column, e.g., via a reflux drum. A Petlyuk system typically comprises a single fractionation column, wherein a pre-fractionation step takes place either in a prefractionation column or, particularly, in a prefractionation section of the fractionation column separated by one or more dividing walls. The latter type is generally referred to as a dividing wall column and includes, among others, so-called dividing top wall columns and dividing mid-wall columns.

Surprisingly, it has been found that passing the methanol content along the top section of a Petlyuk fractionation section results in a methanol fraction of a very pure degree, which does not require further purification. The produced pure grade methanol fraction can for example have a purity of more than 99,9 wt%.

The use of only a single reboiler provides significant economic savings. Energy savings of more than 35% can be obtained, compared with purification using standard distillation columns.

At least the largest part of the methanol content is passed along the top section of the fractionation section. For example, at least 50 wt% of the methanol content, e.g., at least 80 wt% or at least 95 wt% of the methanol content of the separation column feed can be passed along the top section.

In this respect, the top section is the section above the one or more inlets, e.g., the upper 25% or upper 10% of the column.

In a specific embodiment the Petlyuk column installation can comprise a prefractionation column and a fractionation column with a single reboiler. The installation comprises a line for transporting vaporized heavies from the bottom of the prefractionation column to a lower section of the fractionation column, and a return line to return re-condensed heavies from the lower section of the fractionation column to the bottom of the prefractionation column. Similarly, the installation comprises a line for transporting vaporized lights from the top of the prefractionation column to a higher section of the fractionation column, and a return line to return re-condensed lights from the higher section of the fractionation column to the top of the prefractionation column.

Alternatively, the Petlyuk column installation can be a dividing wall column, such as a dividing top wall column or a dividing mid-wall column.

The dividing wall column can for example be a dividing top wall column having at least one dividing wall extending substantially vertically from the top down to an undivided bottom section. The dividing wall separates an inlet side from an outlet side of the top and middle sections of the column. The inlet side forms the prefractionation section. Such a dividing top wall column can for instance have one or more raw grade methanol inlets at the inlet side of the column, at least one heavies outlet for the discharge of heavies and water at the bottom section of the column, at least one lights outlet at the inlet side of the top section of the column and at least one methanol outlet at the outlet side of the top section of the column, e.g., without any lateral methanol outlet.

In one embodiment, the mass flow of methanol through the dividing top wall column can for instance be at least 900 kg/h, e.g., at least 1000 kg/h, or at least 1100 kg/h.

Alternatively, a dividing midwall column can be used having at least one dividing wall extending substantially vertically between an undivided top section and an undivided bottom section. The dividing wall separates an inlet side from an outlet side of the middle sections of the column. The inlet side section forms a prefractionation section. Such a dividing midwall column can for instance have one or more raw grade methanol inlets at the inlet side of the column, at least one heavies outlet for the discharge of heavies and water at the bottom section of the column, at least one lights outlet at the top section of the column and at least one methanol outlet at the top section of the column and/or at the outlet side of a middle section of the column. The distance between the methanol outlets and the top end of the dividing wall may for instance correspond to at least one third or one quarter of the height of the dividing wall. An advantage of a dividing midwall column is that only a single condenser needs to be used, which contributes to further savings in energy and investment costs.

The dividing walls are generally substantially vertical but other orientations can also be used if so desired. Optionally, the dividing wall column may comprise more than one dividing wall.

Good results are obtained when the dividing wall column is operated at or near atmospheric pressure, e.g., with a pressure in the range of about 0.3 - 2 atm, e.g., about 0.6 - 1.5 atm or about 0.8 - 1.2 atm. At 1 atm, temperatures in a dividing top wall column may for example range from 40°C on the top side where lights are withdrawn, to 65°C on the opposite top side where methanol is withdrawn and to 105°C near the bottom where the heavies stream is separated. With a dividing midwall column the temperatures may for example range from 65°C in the top section to 105°C near the bottom where the heavies stream is separated.

In a specific embodiment, the dividing top wall or midwall column may have 10 - 80 theoretical stages, e.g. 25 - 35 stages filled with (structured) packing internals and / or trays. Such packings are solid or hollow bodies of predetermined size, shape and configuration used as column internals to provide surface for liquid to allow mass transfer at the liquid-vapor interface during countercurrent flow of two phases. With a structured packing individual members have a specific orientation relative to each other and to the column axis. Structured packings are usually made of thin metal foil, expanded metal, plain sheet metal, and/or woven wire screen stacked in layers or as spiral bindings, but other packing types can also be used. Trays can be used instead of packings or in addition thereto. Such a tray typically comprises a decking or contacting deck with means to deliver liquid to the tray from a next higher tray and to remove liquid for passage to the next lower tray. The liquid removed from the tray flows down through downcomer of the tray. Vapor generated in a lower portion of the column passes upward through perforations in the decking, while liquid flows downward from tray to tray countercurrent to the vapor.

Particularly suitable are the following types of packing and/or trays: Sulzer Mellapak®(Plus), CY, BX(Plus), I/C/P/R-ring, Pall rings, Cascade MiniRing®, Raschig® rings, Raschig® Super-Ring/Pak, Intalox® (Ultra), Berl® saddles, Nutter® rings, hollow fibers, VGPlus trays, SuperFrac trays, (wire-mesh-packed) sieve trays, bubble cap trays, or valve trays.

The above disclosed purification process is particularly suitable for purifying crude methanol production batches produced by the hydrogenation of carbon dioxide. Such crude grade methanol, to be purified in the dividing wall column, can for instance be produced in a very cost-efficient way by a process comprising the following steps:
- feeding a gas with a H₂:Co₂ ratio between 2.5:1 - 18:1 to a gas-phase catalytic reactor, such as a multi-tubular plug flow reactor;
- in the catalytic reactor the gas is contacted to a catalyst at a temperature of 200 - 300°C and a pressure between 40 - 200 atm to yield a mixture of methanol, carbon monoxide, carbon dioxide and hydrogen.

Optionally, the mixture can subsequently be cooled, e.g. in a feed-effluent heat exchanger and, optionally, subsequently in a reboiler of a stripping column. Methanol and water can then be separated from non-condensable components, thereby forming a methanol product stream and a first recycle gas stream. The methanol product stream can then be stripped using a hydrogen stream, which removes carbon monoxide, carbon dioxide and hydrogen contents and forms a stripped methanol product stream comprising methanol, water, and minor amounts of heavies and lights, and a second recycle stream comprising carbon dioxide and hydrogen. The first and/or second recycle streams can at least partly be returned a feed for the first process step.

Optionally, hydrogen can first be stripped from the crude methanol fraction before the methanol fraction is fed to the dividing wall column. Alternatively, hydrogen can be separated in the dividing wall column, for instance in the top section at the inlet side of a dividing top wall column.

Such a process can for example be carried out in a multi-tubular plug-flow separation column, a fixed bed separation column or a fluidized bed separation column or any other suitable type of separation column.

The required hydrogen feeds can for instance be obtained from a chlorine production plant.

Suitable catalysts are for instance Cu/ZnO based catalysts, such as Cu/ZnO/Al₂O₃/ZrO₂ catalyst, a Cu/ZnO /ZrO₂ /Al₂O₃SiO₂ catalyst or mixtures thereof.

Optionally, the hydrogen carbon dioxide mixture in the first step is heated in a feed-effluent heat-exchanger by the outlet stream of the subsequent step.

Optionally, the hydrogen carbon dioxide mixture in the first step is formed by mixing a depressurized carbon dioxide feed and a hydrogen feed that is pressurized to 30 - 50 atm. Subsequently, the mixture can be pressurized to 50 - 200 atm.

The present invention is further illustrated by the following non-limiting Examples, which are simulations generated with simulation software Alpen Plus®, using RADFRAC® and MULTIFRAC® models for the distillation columns and the Soave modification of Redlich-Kwong equation of state (EOS) and the NRTL liquid activity model with Henry components.

### Example 1

A simulation example was generated based on a dividing midwall column with a common top rectifying section, a split mid section, and a common bottom stripping section. The feed stream consists of 18366 kg/hr mixture consisting of 11581 kg/hr methanol and 6783 kg/hr water, the rest being lights, such as dimethyl ether. The feed is liquid at the temperature of 82°C, and elevated pressure (1,2 atm). The column is operated at 1 atm (condenser level) and has 30 theoretical stages - number from 1 in the top (condenser) to 30 in the bottom (reboiler) - with the dividing-wall extending between stage 18 and stage 24. The feed stream location is at the level of stage 23 at the feed side of the column, also referred to as prefractionator side.

Lights are removed as a gas/vapor distillate from the top of the column. Methanol flows towards the top section of the column over the dividing wall. Part of the methanol is removed as liquid top distillate (3174 kg/hr at over 99.99 %wt purity). The rest of the methanol accumulates at the other side of the dividing wall where another part of the methanol (8393 kg/hr at over 99.99 %wt purity) is collected as a side-draw stream from the side opposite to the feed side of the dividing wall. Water with traces of heavies (6774 kg/hr) is separated as bottom product of the dividing wall column. The total heat consumption in this case is only 6831 kW, which means about 45% energy savings as compared to the conventional direct sequence of two distillation columns.

In the following, a comparison is made between a conventional direct sequence of two distillation columns (Comparative Example), and a dividing top-wall column (Example 2). In both cases, the feed flow rate is 18451 kg/hr containing: 11582 kg/hr methanol, 6783 kg/hr water, the rest being lights. The feed is liquid at the temperature of 32°C and an elevated pressure of 1.2 atm.

### Comparative Example

In the conventional direct sequence of two columns operated at 1 atm (condenser level), the first column has 10 stages (being fed on stage 5), while the second distillation column has 20 stages (with the bottom stream of the first column being fed on stage 15), the respective stages being numbered from 1 in the top (condenser) to 10, 20 in the bottom (reboiler), respectively. The lights are removed as a vapor distillate (86 kg/hr) in the top of the first column using a partial vapor condenser. The bottom product of the first column consists of methanol, water and traces of heavies. The final separation in the second column delivers high purity methanol (over 99.99 %wt) as top distillate, and water (with minor amounts of heavies) as bottom product. The energy consumption of the reboiler was 761 kW for the first column and 12590 kW for the second column, respectively - thus giving a total energy consumption of 13351 kW.

### Example 2

The dividing wall column configuration was a dividing top wall column with a divided overhead section or top section and a common bottom section. The dividing wall column has 30 theoretical stages numbered from 1 in the top (condenser) to 30 in the bottom (reboiler) with the dividing wall located along the top 20 stages (from the top of the column to stage 20), while the feed stream location is on stage 10 (feed side of the column). The lights are removed as gas/vapor from the top of the feed side of the column, while methanol is being removed as top distillate on the (opposite) product side of the column, and water plus heavies are withdrawn from the bottom section. The dividing wall column gives the same performance in terms of purities and recoveries of all products, while having the same total number of theoretical stages as the conventional direct sequence of two columns. However, the total energy consumption with the dividing wall column is only 8429 kW, which means a 36.4% energy savings compared to the conventional direct sequence of two columns. Moreover, as only one reboiler is used and a single shell, this configuration also allows about 25 % reduction of investment costs, as compared to conventional distillation sequences.

The invention will be further explained under reference to the accompanying drawings. In the drawings:
- Figure 1:: shows schematically a dividing top wall column for use in a process according to the present invention;
- Figure 2:: shows schematically a dividing midwall column for use in a process according to the present invention;
- Figure 3:: shows schematically a further alternative column configuration for use in a process according to the present invention.

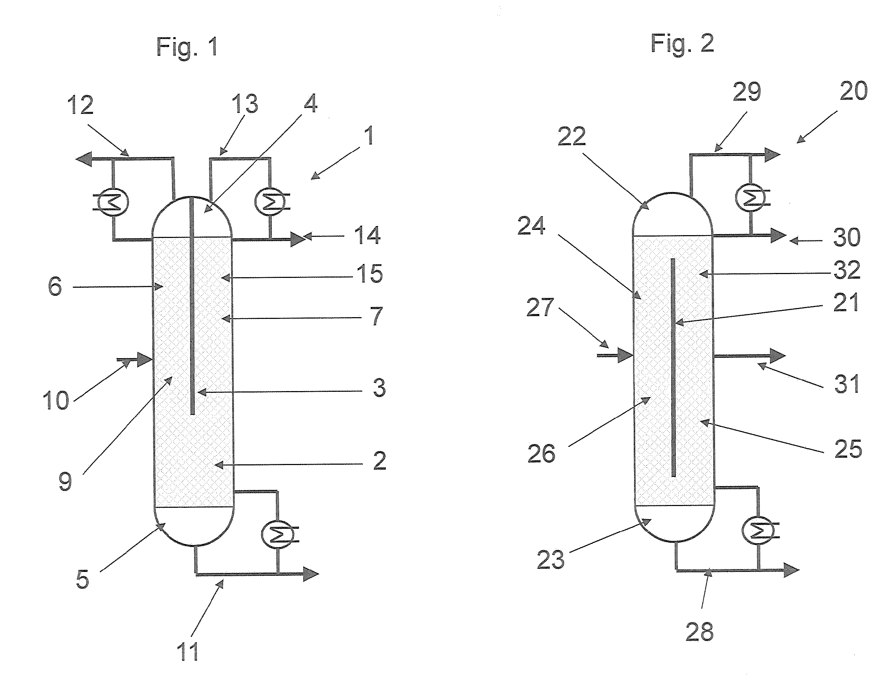

Figure 1 shows a Petlyuk column installation configured as a dividing top wall column 1 with an interior 2 which is divided by a vertical dividing wall 3 extending from the top 4 of the column 1 down to an undivided bottom section 5. The dividing wall 3 separates an inlet side 6 from an outlet side 7 of the top and middle sections 4, 9 of the interior 2. The inlet side 6 forms a prefractionation section, while the outlet side 7 with the bottom section 5 forms a fractionation section. A raw grade methanol inlet 10 is provided at the inlet side 6 of the column 1. A reboiler 11 collects heavies and water from the bottom section 5 of the column 2. The reboiler 11 heats the collected fraction and returns the generated vapors to the column 1.

At the top section 4 the column 1 comprises a first condenser 12 at the inlet side 6 and a second condenser 13 at the outlet side 7. The first condenser 12 condenses collected lights and partly returns the condensed fraction to the column 1. The second condenser 13 condenses collected methanol and also partly returns the condensed fraction to the column 1. A discharge line 14 discharges substantially pure condensed methanol from the condenser 13. The column 1 is not provided with any lateral methanol outlet.

The dividing wall column 1 has internals 15 comprising stacks of packings and or trays. The internals 15 may for example comprise 30 theoretical stages numbered from 1 in the top 4 near the condenser 12 to 30 in the bottom 5 near the reboiler 11, with the dividing wall 3 being located along the twenty highest stages (from the top 4 of the column down to stage 20), while the feed inlet 10 is at the level of stage 10.

Figure 2 shows a dividing midwall column 20 with a vertical dividing wall 21 extending between an undivided top section 22 and an undivided bottom section 23. The dividing wall 21 separates an inlet side 24 from an outlet side 25 of the middle sections 26 of the column. The inlet side 24 of the middle section forms the prefractionation section. The undivided top and bottom sections 22, 23 and the outlet side 25 of the middle section 26 jointly form the fractionation section. The column 20 has a raw grade methanol inlet 27 at the inlet side 24 of the column 20, a reboiler 28 for the discharge of heavies and water at the bottom section 23 of the column 20, a partial condenser 29 at the top section 22 of the column 20, a first methanol outlet 30 at the top section 22 of the column 20 and a second methanol outlet 31 at the outlet side 25 of a middle section 26 of the column 20.

The dividing midwall column 20 contains internals 32 comprising stacks of packings and or trays (not shown). The internals 32 may for example comprise 30 theoretical stages - numbered from number 1 in the top 22 near the condenser 29 to number 30 at the bottom 23 near the reboiler 28, with the dividing-wall extending between stage 18 and stage 24. The raw grade methanol inlet 27 is at the level of stage 23.

When the raw grade methanol enters the column 20, water and heavies flow downwardly towards the bottom section and the reboiler 28. The reboiler 28 collects and heats the mixture of heavies and water and returns the generated vapors to the column 20.

Methanol and light flow upwardly towards the partial condenser 29 at the top section. The condenser 29 condenses the methanol but not the lights, which are discharged as a vapor. Condensed methanol is partly returned to the column 20 and partly discharged via the first methanol outlet 30.

The largest part of the methanol content flows via the top section 22 back down at the outlet side 25 of the column 20 to the main methanol outlet 31, which is at about the same level as the inlet 27.

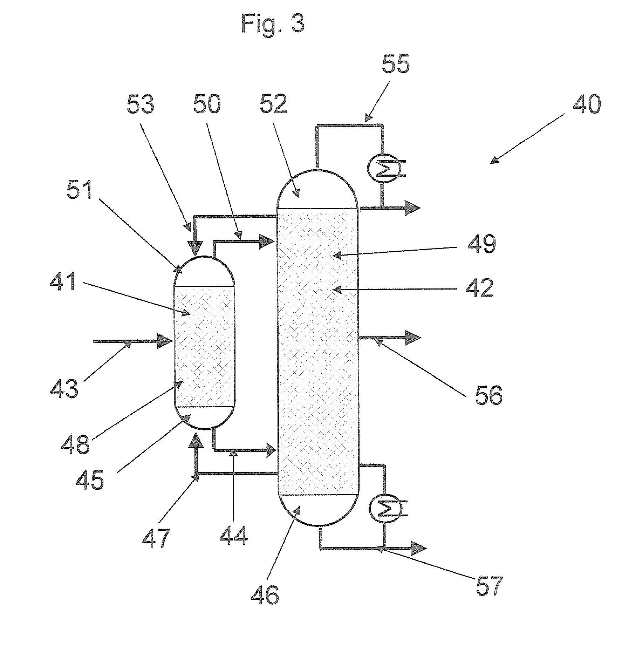

Figure 3 shows a further possible column arrangement comprising a Petlyuk column installation 40 with a prefractionation column 41 and a fractionation column 42. The prefractionation column 41 comprises a feed inlet 43 for the inlet of a crude grade methanol fraction. In the prefractionation column 41 heavies flow down, while lights and methanol flow upwardly.

The prefractionator 41 operates at atmospheric pressure within the temperature range of 70 - 77°C (from top to bottom) and is equipped with common internals 48, such as trays or packing.

A heavies line 44 transports vaporized heavies from the bottom 45 of the prefractionation column 41 to a lower section 46 of the fractionation column 42. A heavies return line 47 returns re-condensed heavies from the lower section 46 of the fractionation column 42 to the bottom 45 of the prefractionation column 41. At the top side a lights line 50 transports vaporized lights from the top 51 of the prefractionation column 41 to a higher section 52 of the fractionation column 42. A lights return line 53 returns re-condensed lights from the higher section 52 of the fractionation column 42 to the top 51 of the prefractionation column 41.

The main fractionation column 42 operates at atmospheric pressure within the temperature range of 65 - 105°C (from top to bottom), and is equipped with common distillation internals 49, such as packing or trays.

In the fractionation column 42 the lights are discharged via a condenser 55 at the top of the fractionation column 42. Here methanol flows down and is discharged via a methanol outlet 56 at about the level of the feed inlet 43.

The fractionation column 42 comprises a reboiler 57 at its bottom 58. The reboiler 57 is a heat exchanger boiling the heavies fraction collected from the bottom 58 of the fractionation column 42 to generate vapors which are returned to the fractionation column 42.

## Claims

1. Process for the purification of methanol wherein a raw grade methanol comprising methanol, lights and heavies is fed to a Petlyuk column installation (1, 20, 40) comprising a fractionation section (7, 25, 42) and a prefractionation section (6, 4, 41) isolated from the fractionation section, wherein the raw grade methanol is fed to the prefractionation section and wherein at least the largest part of the methanol content is passed along a top section (4, 22, 52) of the fractionation section to be subsequently discharged as a pure grade methanol fraction.

2. A process according to claim 1 wherein the Petlyuk column installation is a dividing wall column (1, 20) with an interior partly divided by a dividing wall (1, 20) between an inlet side (6, 24) and an outlet side (7, 25), the inlet side forming the prefractionation section, the remainder of the dividing wall column forming the fractionation section.

3. A process according to claim 2 wherein the dividing wall column is a dividing top wall column (1).

4. A process according to claim 3 wherein the dividing midwall column (1) has 10 - 80 theoretical stages and wherein the dividing wall (3) extends along the upper 30 - 70 % of the stages.

5. A process according to claim 2 wherein the dividing wall column is a dividing midwall column (20).

6. A process according to claim 5 wherein the dividing midwall column (20) has 10 - 80 theoretical stages and wherein the dividing wall (21) extends along about 15 - 80 % of the stages.

7. A process according to claim 1 wherein the Petlyuk column installation (40) comprises a prefractionation column (41) and a fractionation column (42) with a single reboiler (57).

8. A process according to claim 5, 6 or 7 wherein only a single condenser (29) is used.

9. A process according to claim 5 or 6 wherein pure grade methanol fraction is discharged via one or more methanol outlets (31) at a level below the top of the dividing wall (21).

10. A process according to claim 9 wherein the distance between the top end of the dividing wall (21) and a highest methanol outlet (31) below the top of the dividing wall (21) corresponds to at least one quarter, e.g., at least one third of the height of the dividing wall.

11. A process according to any one of the preceding claims wherein the raw grade methanol is first stripped by a hydrogen stream before it is fed to the Petlyuk column installation (1, 20, 40).

12. A process according to any one of the preceding claims wherein the raw grade methanol is at least partly formed by hydrogenation of carbon dioxide.
